# EUROPEAN PATENT APPLICATION

(11) **EP 2 682 381 A2**
(43) Date of publication of application: **08.01.2014**
(21) Application number: 12752744.8
(22) Date of filing: 29.02.2012
(51) Int. Cl.: C07C 229/46, A61K 31/195, A61P 25/18

(54) **SPIROCYCLIC CYCLOPROPANOIC AMINO ACIDS - ANALOGUES OF GAMMA-AMINOBUTYRIC ACID, WITH LIMITED CONFORMATIONAL MOBILITY, AND DRUGS ON THE BASIS THEREOF**

(30) Priority: 03.03.2011 RU 2011108063
(71) Applicant: Zapolsky, Maxim Eduardovich, Moskovskaya obl. 140073 (RU)
(72) Inventor: YASHIN, Nikolay Vladimirovich, Kaluzhskaya obl. 249340 (RU); CHEMAGIN, Andrey Valerievich, Moskovskaya obl. 141100 (RU); KUZNETSOVA, Tamara Stepanovna, Moscow 119192 (RU); ZEFIROV, Nikolay Serafimovich, Moscow 119421 (RU)
(74) Representative: Glawe, Delfs, Moll
(86) International application number: PCT/RU2012/000146
(87) International publication number: WO 2012/118409

(57) **Abstract**

The present invention relates to the field of organic and medicinal chemistry, and may also be used in applied medicine. The essence of the invention consists in that novel spirocyclic cyclopropanoic amino acids of general formula I, which are conformationally limited analogues of γ-aminobutyric acid, are proposed. In formula I, where n= 0-3 and Z=CR⁶R⁷, R¹ - R⁷ are selected independently from a group consisting of hydrogen, (C₁-C₆)-alkyl and cyclo(C₁-C₆)-alkyl. These amino acids exhibit anti-psychotic, antidepressive, anti-spasmodic and anxiolytic properties. Drugs are also proposed for treating psychotic and depressive disorders, including for schizophrenia and affective dysfunctions; for treating convulsive conditions of varying genesis, including within the scope of epilepsy; for treating anxiety disorders of varying genesis, including generalized anxiety dysfunction and panic attacks; for treating somatoform dysfunctions, including persistent pain; for treating pain syndrome of varying genesis; for treating cognitive disorders, including attention and memory disorders of varying genesis.

## Description

### FIELD OF THE INVENTION

The invention relates to novel polyspirocyclic cyclopropanoic amino acids, which are conformationally restricted analogues of natural gamma-aminobutyric acid (GABA) .

### BACKGROUND OF THE INVENTION

GABA is involved in neurotransmitter and metabolic processes in the brain, playing a leading role in pathogenesis of anxiety, seizures and many other pathologic states of the central nervous system (CNS).

GABA_{C} receptors (one of the subclasses of GABA receptors) that are localized in the thalamus, hippocampus and other areas of the brain are responsible for the processes associated with the regulation of sleep and memory. For example, a selective antagonist of the GABA receptor (1,2,5,6-tetrahydropyridine-4-yl)methyl-phosphinic acid (TPMPA) in a number of experiments noticeably improved memory and learning ability of rats and chicks, reduced training time and contributed to a more reliable memorizing of the learned. When studying the effect of TPMPA on sleep it has been found that the application of this substance increases the duration of wakefulness state in rats, while the duration of both slow and paradoxical sleep phase decreases.

Exposure to another antagonist of GABA_{C} and GABA_{B} receptors, known as CGP36742, reduces memory deficit associated with age in rats and enhances cognitive abilities of monkeys.

There are reasons to believe that drugs that affect GABA_{A} subtype alpha 2 can effectively improve working memory in schizophrenia in the comprehensive treatment of this disease (Psychopharmacology (Berl). 2004 Jun;174(1):143-50. Epub 2003 Dec 9. "Selective alterations in prefrontal cortical GABA neurotransmission in schizophrenia: a novel target for the treatment of working memory dysfunction" Lewis DA, Volk DW, Hashimoto T).

The main problem in studying of GABA receptors is the absence of ligands, which combine selectivity towards a particular receptor subtype and high activity.

Currently there are agents being developed that affect GABAergic receptors, which presumably may become selective hypnotics, non-sedative anxiolytics, agents that improve memory, as well as potent analgesics (Neropharmacology. 2010 Oct 28. The rise of a new GABA pharmacology. Möhler H.).

One area of search for such agents is the synthesis of various products of synthetic analogues of GABA.

Cyclopropanoic amino acids are part of the highly effective pharmaceuticals, play an important role in the research of metabolic processes and mechanisms of action of enzymes. Incorporated in the peptide sequence, they alter protein structure and, as a consequence, the biological properties. This is due to the fact that the presence in the molecule of a three-membered ring limits rotation around the C-C bond. Substituents are rigidly fixed in space, but, unlike the unsaturated amino acids, retain asymmetric centres. Incorporation of such acids in the peptides creates a fragment of the sequence that is more stable to hydrolysis. At the present time an up to date direction in the field of medical and organic chemistry is the search for novel conformationally rigid analogues of natural amino acids. Cyclopropanoic amino acids in some cases possess a more selective effect on receptor systems than their corresponding natural analogues.

Closest to the proposed with respect to technical essence are analogues of glutamic acid - 1-aminospiro[2.2]pentane-1,4-dicarboxylic acid [Proceedings of the Academy of Sciences, Volume 419, N° 6, April 2008, pp. 772-774] and 1-aminospiro[3.2]hexane-1,5-dicarboxylic acid [XI International Scientific and Technical Conference "Perspectives of chemistry development and practical application of the alicyclic compounds", Volgograd, June 3-6, 2008], the synthesis of which is based on the reactions of [1+2] cycloaddition of ethyl nitrodiazoacetate to methyl ethers of 2-(methylene) cyclobutanecarboxylic acid and 3-(methylene)-cyclopropanecarboxylic acid, leading to formation of 1-ethyl-4-methyl-1-nitrospiro[2.2]pentane-1,4-dicarboxylate (hereinafter adduct **1**) and 1-ethyl-5-methyl-1-nitrospiro[2.3]hexane-1,5-dicarboxylate (hereinafter adduct **2**), followed by successive reduction of the nitro group and hydrolysis of the ethoxycarbonyl substituents in adducts **1** and **2**.

However, these amino acids are not of interest as conformationally restricted analogues of the gamma-aminobutyric acid that are suitable for clinical use because the presence of an additional carboxylic fragment in alpha-position to the amino group within the ligand molecule is inadmissible for the successful binding to GABA receptors.

### DISCLOSURE OF THE INVENTION

The goal of the present invention is to provide novel spirocyclic cyclopropanoic amino acids, which are of interest in the capacity of conformationally restricted analogues of gamma-aminobutyric acid and which may find use in the applied medicine.

This goal is achieved by a compound, which is a cyclopropanoic amino acid of the general formula **I**, which is a potential ligand of gamma-aminobutyric acid receptors (hereinafter GABA-receptors) and which can find use in applied medicine as in the capacity of an anxiolytic drug. 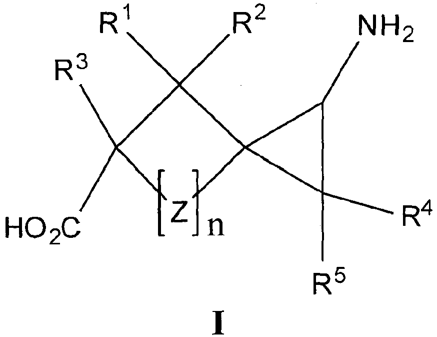
wherein n=0-3,
Z=CR⁶R⁷,
R¹ - R⁷ are independently selected from the group comprising: hydrogen,
(C₁ - C₆)-alkyl, cyclo (C₁ - C₆) -alkyl .

The method of synthesis of novel compounds is in that a selective hydrolysis and decarboxylation of one of the two carboxyl groups being in alpha-position to the nitro group are carried out in the adducts **1** and **2** obtained at the stage of addition of ethyl nitrodiazoacetate to methyl ethers of 2-(methylene) cyclopropanecarboxylic acid and 3-(methylene) cyclobutanecarboxylic acid, followed by a reduction of nitro group to amino group and a hydrolysis of the second carboxyl moiety to form target amino acids of the general formula **I**.

The claimed compounds can be used as an individual compound or as part of pharmaceutical compositions comprising a pharmaceutical carrier in different pharmaceutical forms. Individual examples of pharmaceutical forms are tablets, capsules, powders, aqueous and non-aqueous solutions and suspensions for oral administration or parenteral administration comprising either one or some larger number of standard dosages, which can be subdivided into individual doses.

Examples of suitable pharmaceutical carriers are sugars such as lactose and sucrose; starches such as corn starch and potato starch; cellulose derivatives such as sodium carboxymethyl cellulose, ethyl cellulose, methyl cellulose and cellulose acetate phthalate; gelatine; talc; stearic acid or its salts; vegetable oils such as sunflower oil, olive oil, corn oil, cocoa butter; various alcohols such as propylene glycol, glycerine; sorbitol; polyethylene glycol; water; pectins; alginic acid or its salts; isotonic sodium chloride solution, phosphate buffer solutions, as well as other acceptable excipients commonly used in pharmaceutical formulations. Compositions may also comprise other components such as colorants, flavours and / or preservatives. Compositions may, if desired, also comprise other therapeutic agents commonly used for the treatment of a treatable disorder or condition.

### BEST EXAMPLE OF IMPLEMENTATION OF THE INVENTION

Below are provided the examples of the realization of the invention.

### Example 1. Synthesis of 4-aminospiro[2.2]pentanecarboxylic acid

### Methyl 4-nitrospiro[2.2]pentane-1-carboxylate

To a solution of 520 mg (2.14 mmol) of diester **3** [3] in 2.5 ml of absolute methanol while stirring 2.5 ml of 1 N sodium hydroxide in methanol was added and stirred for 40 min. The solvent was evaporated under reduced pressure, the remaining residue was dissolved in 5.5 ml of dimethyl sulfoxide-water - 10:1 (*v* / *v*) and heated for 30 min on oil bath at temperature of 80 °C. The reaction mixture was cooled to room temperature and diluted with an equal volume of water, extracted with 4x15 ml of diethyl ether. The organic layer was dried over magnesium sulfate, the solvent was evaporated under reduced pressure, the remaining residue was further purified by preparative column chromatography on silica gel, eluent petroleum ether-ethyl aceate - 95:1. The 304 mg of the final nitroester was recovered as colourless oil. Yield 83%.

NMR ¹H (CDCl₃, δ, ppm) for a mixture of two isomers: 0.63-0.69 (m, 1H+1H, c-Pr-CH₂), 0.98-1.11 (m, 1H+1H, c-Pr-CH₂), 1.30-1.39 (m, 1H+1H, c-Pr-CH₂), 1.90 (br s, 2H+2H, NH₂), 2.35-2.39 (m, 1H+1H, c-Pr-CH₂), 2.53 -2.58 (m, 1H+1H, c-Pr-CH), 3.56 (s, 3H+3H, OCH₃), 3.84-3.90 (m, 1H+1H, c-Pr-CH).

### 4-aminospiro[2.2]pentanecarboxylic acid

The 1520 mg (23.4 mmol) of zinc powder was added to a solution of 200 mg (1.17 mmol) of methyl 4-nitrospiro[2.2]pentanecarboxylate and 702 mg (0.67 ml, 11.7 mmol) of glacial acetic acid in 23 ml of isopropanol at room temperature in small portions over 30 min. The mixture was stirred for 6 h, treated with saturated sodium hydrogen carbonate up to pH 8. The precipitate was filtered off, the filtrate was extracted with CH₂Cl₂ (4x5 ml). The extract was washed with water (4x5 ml), dried over magnesium sulfate, the solvent was evaporated. A solution of 78 mg (2.0 mmol) of sodium hydroxide in 1.9 ml of methanol was added to the remaining residue (110 mg). The reaction mixture was stirred at room temperature for 48 hrs, then 0.2 M hydrochloric acid solution was added up to pH 3, the solvent was evaporated under reduced pressure, the remaining residue was dissolved in 4 ml of distilled water and subjected to chromatography (Dowex 50, eluent - 0.9 M aqueous solution of ammonia). The solvent was evaporated and the remaining residue was re-crystallized from ethanol-water (1:1) system. The 93 mg of 4-aminospiro[2.2]pentanecarboxylic acid was obtained as pale-yellow crystals. Yield 62%.

NMR ¹H (CD₃OD, δ, ppm) for a mixture of two isomers: 1.41-1.54 (m, 2H+2H, c-Pr-CH₂), 1.56-1.61 (m, 1H+1H, c-Pr-CH₂), 1.70-1.75 (m, 1H+1H, c-Pr-CH₂), 2.26-2.37 (m, 1H+1H, c-Pr-CH), 3.11-3.20 (m, 1H+1H, c-Pr-CH).

### Example 2. Synthesis of 1-aminospiro[2.3]hexane-5-carboxylic acid

### Methyl 1-nitrospiro[2.3]hexane-5-carboxylate

To a solution of 550 mg (2.14 mmol) of diester **4** [2] in 2.5 ml of absolute methyl alcohol while stirring 2.5 ml of 1 N sodium hydroxide in methanol was added and stirred for 40 min. The solvent was evaporated under reduced pressure, the remaining residue was dissolved in 5.5 ml of the mixture of dimethyl sulfoxide-water - 10:1 (*v* / *v*) and heated for 30 minutes on oil bath at temperature of 80 °C. The reaction mixture was cooled to room temperature and diluted with an equal volume of water, extracted with 3x20 ml of diethyl ether. The organic layer was dried over magnesium sulfate, the solvent evaporated under reduced pressure, the remaining residue was further purified by preparative column chromatography on silica gel, eluent petroleum ether-ethyl aceate - 95:1. The 310 mg of the final nitroester was recovered as colourless oil. Yield 78%.
NMR ¹H (CDCl₃, δ, ppm) for a mixture of two isomers: 0.43-0.49 (m, 1H+1H, c-Pr-CH₂, isomers **A** and **B**), 1.09-1.16 (m, 1H+1H, c-Pr-CH₂, isomers **A** and **B**), 1.92-2.08 (m, 2H+2H, c-Bu-CH₂, isomers **A** and **B**), 2.15-2.25 (m, 2H+2H, c-Bu-CH₂, isomers **A** and **B**), 2.30-2.34 (m, 1H+1H, c-Pr-CH, isomers **A** and **B**), 3.02-3.10 (m, 1H+1H, c-Bu-CH, isomers **A** and **B**), 3.51 (s, 3H+3H, OCH₃, isomers **A** and **B**).

### 1-Aminospiro[2.3]hexane-5-carboxylic acid

The 1400 mg of zinc powder (21.5 mmol) was added to a solution of 200 mg (1.08 mmol) of methyl 1-nitrospiro[3.2]hexane-5-carboxylate and 650 mg (0.62 ml, 10.8 mmol) of glacial acetic acid in 22 ml of isopropanol at room temperature in small portions over 30 min. The mixture was stirred for 6 h, treated with saturated solution of sodium hydrogen carbonate up to pH 8. The precipitate was filtered off, the filtrate was extracted with CH₂Cl₂ (4x5 ml). The extract was washed with water (4x5 ml), dried over magnesium sulfate, the solvent was evaporated. A solution of 80 mg (2.0 mmol) of sodium hydroxide in 2 ml of methanol was added to the remaining residue (125 mg). The reaction mixture was stirred at room temperature for 48 hrs, then 0.2 M hydrochloric acid solution was added up to pH 3, the solvent was evaporated under reduced pressure, the remaining residue was dissolved in 4 ml of distilled water and subjected to chromatography (Dowex 50, eluent - 0.9 M aqueous solution of ammonia). The solvent was evaporated and the remaining residue was re-crystallized from ethanol-water (1:1) system. The 108 mg of 1-aminospiro[2.3]hexane-5-carboxylic acid was obtained as pale-yellow crystals. Yield 71%.
NMR ¹H (CD₃OD, δ, ppm) for a mixture of two isomers: 0.40-0.45 (dd, ²*J*_{H},_{H} = 5.6 Hz, ³*J*_{H},_{H} = 4.3 Hz, 1H+1H, c-Pr-CH₂), 0.89 -0.96 (dd, ²*J*_{H},_{H}= 7.3 Hz, ³*J*_{H},_{H} = 6.1 Hz, 1H+1H, c-Pr-CH₂), 1.96-2.09 (m, 2H+2H, c-Bu-CH₂), 2.17-2.30 (m, 2H+2H+1H, c-Bu-CH₂ + c-Pr-CH), 2.30-2.41 (m, 1H, c-Pr-CH), 3.05-3.11 (m, 1H+1H, c-Bu-CH).

### Example 3. The study of the psychoactive properties

Experiments were carried out on mature male rats weighing 180-200 g, grown in the breeding nursery of RAS "Rappolovo". Before experiments animals were subjected to 14 day quarantine and randomization. Tags and individual numbers of animals were recorded in the protocols of the laboratory tests.

### Evaluation of anxiolytic activity on the model of aggressive behaviour of the pair of rats, which is provoked by an electro-painful stimulation through the electrical floor

Aggressive responses in rats were induced by supramaximal electrical stimulation of the floor on the pair of rats (stimulation by the electric current of 1.5 mA). Criterion of the aggressive response was a "fight" of the pair of animals in standing on hind legs for 1-2 minutes. For each dose of tested substance a group of 6 pairs of animals was used.

The tested substances of Example 1 and Example 2 were administered to experimental animals intraperitoneally at doses of 0.1, 1, 10 mg / kg 40 minutes before the experiment. Pharmacopoeial substance Phenazepam - 1 mg / kg was used as a reference drug. The control group of rats received water for injections (intraperitoneally - 1 ml / kg) .

The results of the experiments are presented in Table 1.

**Table 1.**

| **Group/compound** | **Dose mg/kg** | **Threshold of aggressiveness** | |
|---|---|---|---|
| | | **M±m, seconds** | **% to control** |
| **Control (water for injections)** | 1 ml/kg | 30.5±2.3 | 100 |
| **Phenazepam** | 1.0 | 58.3±4,1 | 191 |
| **Example 1** | 0.1 | 35.5±2.8* | 116 |
| | 1.0 | 47.8±3.3 | 157 |
| | 10 | 59.1±3.4 | 194 |
| **Example 2** | 0.1 | 61.3±8.9 | 201 |
| | 1.0 | 50.2±3.5 | 165 |
| | 10 | 69.1±5.7 | 227 |

| | | | |
|---|---|---|---|
| *Note:* * - *differences from the measurements of the control group of animals were not statistically significant p≥0.05.* | | | |

It was found that intraperitoneal administration of the compound of Example 1 at a dose of 0.1 mg / kg to rats statistically increased the threshold of aggressiveness by 19% compared to the measurement of the control group of animals. In case of administration of a higher dose (10 mg / kg) to the experimental animals changes of the rats' threshold of aggressiveness were comparable with the measurement of the threshold of aggressiveness of the animals treated with the reference drug - Phenazepam (1 mg / kg).

When the compound of Example 2 was administered intraperitoneally to the experimental animals its pronounced antiaggressive action was observed in all tested doses. This was reflected by a two fold increase of the aggressiveness thresholds of rats in case of the administration of the compound at doses of 0.1 mg / kg and 10 mg / k to them in comparison with the measurement of the control group of animals. In these doses the claimed compound surpassed the antiaggressive effect of Phenazepam at 1 mg / kg.

When the compound of Example 2 was administered intraperitoneally to the experimental animals at a dose of 1.0 mg / kg the aggressiveness threshold was increased by 65% compared with the control group of animals and was somewhat inferior to the reference drug - pharmacopoeial substance Phenazepam.

As seen from the experiments the compounds have the proven psychoactive activity.

### INDUSTRIAL APPLICABILITY

Novel spirocyclic cyclopropanoic amino acids as proposed in the present invention are of interest in the capacity of conformationally restricted analogues of gamma-aminobutyric acid that may find use in the applied medicine as an anxiolytic drug.

## Claims

1. Spirocyclic cyclopropanoic amino acids analogues of gamma-aminobutyric acid with restricted conformational mobility having the general formula **I** 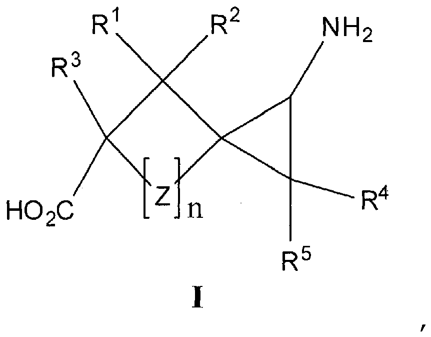
wherein n=0-3,
Z=CR⁶R⁷,
R¹ - R⁷ are independently selected from the group comprising: hydrogen, (C₁ - C₆) -alkyl, cyclo (C₁ - C₆) - alkyl.

2. Spirocyclic cyclopropanoic amino acids of claim 1 exhibiting antipsychotic, antidepressive, anticonvulsive, anxiolytic properties.

3. Medicament for the treatment of psychotic and depressive disorders including schizophrenia and affective disorders.

4. Medicament for the treatment of convulsions of various origins including epilepsy framework.

5. Medicament for the treatment of anxiety disorders of various origins including generalized anxiety disorder, panic attacks.

6. Medicament for the treatment of somatoform disorders including intractable pain.

7. Medicament for treatment of pain syndrome of various origins.

8. Medicament for the treatment of cognitive disorders including attention and memory disorders of various origins.
